# EUROPEAN PATENT APPLICATION

(11) **EP 4 803 091 A1**
(43) Date of publication of application: **09.09.2026**
(21) Application number: 24886163.5
(22) Date of filing: 28.10.2024
(51) Int. Cl.: A61K 35/747, A61P 43/00, A61P 29/00, A61P 1/12, A23L 33/135

(54) **COMPOSITION FOR PREVENTING, ALLEVIATING OR TREATING CACHEXIA, COMPRISING LACTOBACILLUS REUTERI ATG-F4 STRAIN**

(30) Priority: 03.11.2023 KR 20230151043
(71) Applicant: Atogen Co., Ltd., Daejeon 34015 (KR); Kang, Jihee, Daejeon 34010 (KR)
(72) Inventor: LEE, Daeyoung, Daejeon 34022 (KR); AN, Dami, Daejeon 34016 (KR); CHO, Subin, Daejeon 34016 (KR); JANG, Eunhye, Daejeon 34822 (KR); IM, Sunghoon, Sejong 30101 (KR); SONG, Wonho, Sejong 30151 (KR); KANG, Jihee, Daejeon 34010 (KR)
(74) Representative: Witte, Weller & Partner Patentanwälte mbB
(86) International application number: PCT/KR2024/016494
(87) International publication number: WO 2025/095489

(57) **Abstract**

The present invention relates to a composition containing a Lactobacillus reuteri ATG-F4 strain as an active ingredient for preventing, alleviating, or treating side effects of cachexia or chemotherapy.

## Description

### [Technical Field]

The present disclosure relates to a composition for preventing, alleviating, or treating cachexia, comprising Lactobacillus reuteri ATG-F4 strain. In particular, the present disclosure relates to a composition comprising Lactobacillus reuteri ATG-F4 strain as an active ingredient and capable of being used for preventing, alleviating, or treating cachexia or side effects of anticancer chemotherapy.

### [Background Art]

In general, in cancer patients, a multifactorial syndrome characterized by an ongoing loss of skeletal muscle mass, a condition that is not reversible by conventional nutritional support, and progressive functional impairment is referred to as cancer cachexia and anorexia.

Unlike patients with other chronic diseases, cancer patients are characterized in that they suffer not only from cachexia but also from side effects of various therapies used to treat cancer. In particular, in the case of head and neck cancer and gastrointestinal cancer, because the affected organs are organs through which food passes or organs responsible for digestion and absorption, patients experience cachexia more frequently than in cancers occurring in other sites, and according to some reports, 50% to 80% of patients experience cachexia, and the mortality rate due to cachexia also reaches 20% to 30%.

Although cancer cachexia is accompanied by weight loss similar to sarcopenia caused by aging or weight loss observed during temporary fasting, it is characterized in that inflammatory responses induced by various cytokines and changes in carbohydrate, protein, and fat metabolism increase catabolic reactions, thereby causing muscle loss, and weight loss occurs despite normal food intake. These changes, together with restricted nutritional intake caused by treatments such as anticancer chemotherapy, radiotherapy, or surgery, lower the response rate to treatment and make it difficult to carry out effective treatment, and consequently may become a major cause of reduced survival rate and quality of life of patients.

Accordingly, appropriate understanding and correction of cachexia increase compliance with treatment, thereby ultimately improving quality of life and alleviating secondary side effects.

Anorexia or weight loss, which cancer patients experience relatively frequently, may occur not only due to cachexia but also as side effects caused by surgery, anticancer chemotherapy, radiotherapy, and the like. However, unlike a general fasting state, cachexia proceeds through a completely different mechanism due to changes in body metabolism, cytokines, and various substances secreted from patients and tumors that are involved in appetite mechanisms, and cachexia resulting therefrom negatively affects the quality of life, survival rate, and prognosis of patients. Although various drugs have been studied for the treatment of cachexia, satisfactory results have not yet been achieved, and thus prospective studies involving a larger number of patients are required in the future.

Accordingly, while conducting various studies on methods for alleviating cachexia by using lactic acid bacteria, the present inventors confirmed that Lactobacillus reuteri ATG-F4 has an excellent effect on the alleviation and treatment of cachexia, thereby completing the present disclosure.

### [Disclosure]

### [Technical Problem]

An objective of the present disclosure is to provide a composition for preventing, alleviating, or treating cachexia, comprising Lactobacillus reuteri ATG-F4 as an active ingredient.

Another objective of the present disclosure is to provide a pharmaceutical composition or a food composition for preventing, alleviating, or treating cachexia, comprising Lactobacillus reuteri ATG-F4 as an active ingredient.

Still another objective of the present disclosure is to provide a composition for preventing, alleviating, or treating side effects of anticancer chemotherapy, comprising Lactobacillus reuteri ATG-F4 as an active ingredient.

The technical problem to be solved by the present disclosure is not limited to the above-mentioned problem(s), and other problem(s) not mentioned herein will be clearly understood by those skilled in the art from the following description.

### [Technical Solution]

In order to achieve the above objectives, the present disclosure provides a composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

In addition, the present disclosure provides a pharmaceutical composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

In addition, the present disclosure provides a food composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

In addition, the present disclosure provides a composition for preventing, alleviating, or treating side effects of anticancer chemotherapy, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

The composition may increase a survival rate reduced by administration of an anticancer agent.

The composition may increase a fat mass reduced by administration of an anticancer agent.

The composition may increase grip strength reduced by administration of an anticancer agent.

The composition may reduce inflammation increased by administration of an anticancer agent.

The composition may inhibit phosphorylation of NF-κB (nuclear factor-κB), which is an inflammation-related factor increased by administration of an anticancer agent.

The composition may reduce damage to colon tissue increased by administration of an anticancer agent.

The composition may restore an expression level of occludin or claudin protein in colon tissue reduced by administration of an anticancer agent.

The composition may reduce a blood concentration of TNF-α or IL-6 increased by administration of an anticancer agent.

The composition may reduce blood lipopolysaccharide (LPS) increased by administration of an anticancer agent.

The composition may reduce symptoms of diarrhea caused by administration of an anticancer agent.

### [Advantageous Effects]

The composition comprising Lactobacillus reuteri ATG-F4 strain according to the present disclosure is effective for preventing, alleviating, or treating cachexia or side effects of anticancer chemotherapy.

In addition, the composition comprising Lactobacillus reuteri ATG-F4 strain according to the present disclosure has an effect of increasing a survival rate reduced by administration of an anticancer agent.

In addition, the composition comprising Lactobacillus reuteri ATG-F4 strain according to the present disclosure has an effect of inhibiting weight loss and decreases in muscle mass and fat mass caused by administration of an anticancer agent.

In addition, the composition comprising Lactobacillus reuteri ATG-F4 strain according to the present disclosure has effects of increasing atrophied muscle fibers caused by administration of an anticancer agent, inhibiting phosphorylation of NF-κB, reducing the muscle atrophy-related protein MuRF1, and increasing mitochondria-related proteins PGC-1α, NRF-1, and mtTFA.

In addition, the composition comprising Lactobacillus reuteri ATG-F4 strain according to the present disclosure has an effect of alleviating damage to colon tissue caused by administration of an anticancer agent.

In addition, the composition comprising Lactobacillus reuteri ATG-F4 strain according to the present disclosure has an effect of restoring an expression level of occludin or claudin protein in colon tissue reduced by administration of an anticancer agent.

In addition, the composition comprising Lactobacillus reuteri ATG-F4 strain according to the present disclosure has an effect of reducing a blood concentration of TNF-α or IL-6 increased by administration of an anticancer agent.

In addition, the composition comprising Lactobacillus reuteri ATG-F4 strain according to the present disclosure has an effect of reducing blood lipopolysaccharide (LPS) increased by administration of an anticancer agent.

In addition, the composition comprising Lactobacillus reuteri ATG-F4 strain according to the present disclosure has an effect of reducing symptoms of diarrhea caused by administration of an anticancer agent.

### [Description of Drawings]

FIG. 1 illustrates a survival rate after administration of Lactobacillus reuteri ATG-F4 (low, medium, and high doses) for 5-FU-induced side effects of anticancer chemotherapy according to an exemplary embodiment of the present disclosure.
FIG. 2a is a graph showing tumor weight after administration of Lactobacillus reuteri ATG-F4 (low, medium, and high doses) for 5-FU-induced side effects of anticancer chemotherapy according to an exemplary embodiment of the present disclosure.
FIG. 2b is a graph showing body weight minus tumor weight after administration of Lactobacillus reuteri ATG-F4 (low, medium, and high doses) for 5-FU-induced side effects of anticancer chemotherapy according to an exemplary embodiment of the present disclosure.
FIG. 2c is a graph showing changes in body weight without tumor for 17 days after administration of Lactobacillus reuteri ATG-F4 (low, medium, and high doses) for 5-FU-induced side effects of anticancer chemotherapy according to an exemplary embodiment of the present disclosure.
FIG. 2d is a graph showing changes in right and left calf muscle weight after administration of Lactobacillus reuteri ATG-F4 (low, medium, and high doses) for 5-FU-induced side effects of anticancer chemotherapy according to an exemplary embodiment of the present disclosure.
FIG. 2e is a graph showing changes in fat weight for 17 days after administration of Lactobacillus reuteri ATG-F4 (low, medium, and high doses) for 5-FU-induced side effects of anticancer chemotherapy according to an exemplary embodiment of the present disclosure.
FIG. 2f is a graph showing changes in grip strength normalized to body weight after administration of Lactobacillus reuteri ATG-F4 (low, medium, and high doses) for 5-FU-induced side effects of anticancer chemotherapy according to an exemplary embodiment of the present disclosure.
The 5-FU was administered at 50 mg/kg, and the low, medium, and high doses of Lactobacillus reuteri ATG-F4 were administered at 1.0 x 10⁸, 1.0 x 10⁹, and 1.0 x 10¹⁰ CFU/day, respectively (one-way ANOVA Dunnett's p<0.001:***, p<0.01:**, p<0.05:* vs LLC_5-FU).
FIG. 3a is a photograph of quadriceps femoris (QF) muscle fibers after administration of Lactobacillus reuteri ATG-F4 (low, medium, and high doses) for 5-FU-induced side effects of anticancer chemotherapy according to an exemplary embodiment of the present disclosure.
FIG. 3b is a graph showing quadriceps femoris (QF) muscle fiber size after administration of Lactobacillus reuteri ATG-F4 (low, medium, and high doses) for 5-FU-induced side effects of anticancer chemotherapy according to an exemplary embodiment of the present disclosure (one-way ANOVA Dunnett's p<0.001:***, p<0.01:**: vs LLC_5-FU).
FIG. 3c is a graph showing expression of inflammation-related proteins (p-NF-κB/NF-κB) in QF muscle after administration of Lactobacillus reuteri ATG-F4 (high dose) for 5-FU-induced side effects of anticancer chemotherapy according to an exemplary embodiment of the present disclosure.
FIG. 3d is a graph showing expression of a muscle atrophy-related protein (MuRF1) after administration of Lactobacillus reuteri ATG-F4 (high dose) for 5-FU-induced side effects of anticancer chemotherapy according to an exemplary embodiment of the present disclosure.
FIG. 3e is a graph showing expression of mitochondria-related proteins PGC-1α, NRF-1, and mtTFA after administration of Lactobacillus reuteri ATG-F4 (high dose) for 5-FU-induced side effects of anticancer chemotherapy according to an exemplary embodiment of the present disclosure.
The 5-FU was administered at 30 mg/kg, and the low, medium, and high doses of Lactobacillus reuteri ATG-F4 were administered at 4.0 x 10⁸, 4.0 x 10⁹, and 4.0 x 10¹⁰ CFU/day, respectively (FIGS. 3c to 3e: one-way ANOVA Dunnett's p<0.001:***, p<0.01:**, p<0.05:* vs LLC_5-FU).
FIG. 4a shows H&E-stained images of colon tissue after administration of Lactobacillus reuteri ATG-F4 (low, medium, and high doses) for 5-FU-induced side effects of anticancer chemotherapy according to an exemplary embodiment of the present disclosure.
FIG. 4b is a graph comparing histological scores of colon tissue after administration of Lactobacillus reuteri ATG-F4 (low, medium, and high doses) for 5-FU-induced side effects of anticancer chemotherapy according to an exemplary embodiment of the present disclosure.
FIG. 4c is a graph showing expression levels of occludin and claudin proteins after administration of Lactobacillus reuteri ATG-F4 (high dose) for 5-FU-induced side effects of anticancer chemotherapy according to an exemplary embodiment of the present disclosure.
FIG. 5a is a graph showing blood TNF-α concentration after administration of Lactobacillus reuteri ATG-F4 (low, medium, and high doses) for 5-FU-induced side effects of anticancer chemotherapy according to an exemplary embodiment of the present disclosure.
FIG. 5b is a graph showing blood IL-6 concentration after administration of Lactobacillus reuteri ATG-F4 (low, medium, and high doses) for 5-FU-induced side effects of anticancer chemotherapy according to an exemplary embodiment of the present disclosure.
FIG. 5c is a graph showing blood endotoxin [lipopolysaccharide (LPS)] concentration after administration of Lactobacillus reuteri ATG-F4 (high dose) for 5-FU-induced side effects of anticancer chemotherapy according to an exemplary embodiment of the present disclosure.
FIG. 6a illustrates a survival rate after administration of Lactobacillus reuteri ATG-F4 for FOLFIRI-induced side effects of anticancer chemotherapy according to an exemplary embodiment of the present disclosure.
FIG. 6b illustrates a survival rate after administration of Lactobacillus reuteri ATG-F4 for FOLFOX-induced side effects of anticancer chemotherapy according to an exemplary embodiment of the present disclosure.
FIG. 7a is a graph showing stool consistency score results after administration of Lactobacillus reuteri ATG-F4 for FOLFIRI-induced side effects of anticancer chemotherapy according to an exemplary embodiment of the present disclosure.
FIG. 7b is a graph showing stool consistency score results after administration of Lactobacillus reuteri ATG-F4 for FOLFOX-induced side effects of anticancer chemotherapy according to an exemplary embodiment of the present disclosure.
FIG. 8 illustrates an experimental schedule for evaluating survival rate according to administration of Lactobacillus reuteri ATG-F4 for 5-FU-induced cachexia symptoms according to an exemplary embodiment of the present disclosure.
FIG. 9 illustrates an experimental schedule for efficacy evaluation according to administration of Lactobacillus reuteri ATG-F4 for 5-FU-induced cachexia symptoms according to an exemplary embodiment of the present disclosure.
FIG. 10 illustrates an experimental schedule for evaluating survival rate according to administration of Lactobacillus reuteri ATG-F4 for FOLFIRI- and FOLFOX-induced cachexia symptoms according to an exemplary embodiment of the present disclosure.

### [Mode for Invention]

In the following description, it should be noted that only those parts necessary for understanding exemplary embodiments of the present disclosure are described, and descriptions of other parts will be omitted insofar as they do not obscure the gist of the present disclosure.

The terms or words used in the present specification and claims described below should not be construed as being limited to ordinary or dictionary meanings, but should be construed as having meanings and concepts consistent with the technical spirit of the present disclosure, based on the principle that the inventor may appropriately define the concepts of terms in order to describe his or her own invention in the best manner.

Accordingly, the exemplary embodiments described in the present specification and the configurations illustrated in the drawings are merely preferred exemplary embodiments of the present disclosure and do not represent all of the technical spirit of the present disclosure, and therefore it should be understood that various equivalents and modifications capable of replacing them may be made at the time of filing the present application.

Hereinafter, the present disclosure will be described in detail.

In the present disclosure, the term "pharmaceutical composition" refers to an agent used for the purpose of diagnosing, treating, alleviating, managing, or preventing a disease in an animal, including a human.

In the present disclosure, the term "food composition" includes fresh foods distributed as they are, such as agricultural, livestock, forestry, and fishery products, and processed foods prepared or processed from raw materials thereof to improve storability, nutritional value, and the like.

In addition, the "food composition" of the present disclosure includes a "health functional food composition," and the term "health functional food composition" means a food manufactured or processed using raw materials or ingredients having functionality useful to the human body, and includes all forms such as functional food, nutritional supplement, health food, and food additive.

The present disclosure provides a composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

In addition, the present disclosure provides a pharmaceutical composition for preventing, alleviating, or treating cachexia, comprising, as an active ingredient, at least one selected from the group consisting of a strain of Lactobacillus reuteri ATG-F4 (Lactobacillus reuteri ATG-F4, accession No. KCTC13717BP), cells of the strain, a culture of the strain, an extract of the culture, a concentrate of the culture, and a dried product of the culture.

In addition, the present disclosure provides a food composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

In addition, the present disclosure provides a composition for preventing, alleviating, or treating side effects of anticancer chemotherapy, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

Physiological/genetic information on the Lactobacillus reuteri ATG-F4 *(Lactobacillus reuteri* ATG-F4, accession No. KCTC13717BP) strain is described in Korean Patent No. 10-1951919 (title of the invention: Novel Lactobacillus reuteri ATG-F4 strain having a dopamine secretion-promoting function, and composition comprising the same for preventing or treating a mental disorder; applicant: ATOGEN CO., LTD.; registration date: February 19, 2019).

In the present disclosure, the term "culture of the strain" means to include the culture broth itself obtained by culturing in a medium, for example, a liquid medium, and a supernatant (filtrate) obtained by removing the strain from the culture broth by filtration and/or centrifugation.

In the present disclosure, the term "extract of the culture, concentrate of the culture, and dried product of the culture" is not limited thereto, although a centrifugation or filtration process may be performed in order to remove the liquid culture medium from the culture and recover only concentrated cells. In addition, the concentrated cells may be preserved without losing activity by drying, freezing, or freeze-drying according to a conventional method.

The strain of Lactobacillus reuteri ATG-F4, cells of the strain, a culture of the strain, an extract of the culture, a concentrate of the culture, and a dried product of the culture may be added to the composition of the present disclosure in an amount of 0.001 to 100 wt%.

In the present disclosure, "cachexia" refers to severe systemic wasting symptoms observed in terminal stages of cancer, tuberculosis, diabetes, acquired immunodeficiency syndrome (AIDS), and the like, and frequently appears in patients with digestive cancers such as gastric cancer, esophageal cancer, pancreatic cancer, and colorectal cancer, as well as in patients with lung cancer.

Cachexia exhibits symptoms such as decreased appetite, reductions in body weight and physical strength due to loss of muscle and fat, anemia, lethargy, and indigestion, and in particular indicates a state in which body weight decreases even though food is consumed normally. When cachexia occurs, patients show a low response to anticancer chemotherapy or radiotherapy, resulting in deterioration of the patient's quality of life, shortening of life expectancy, and death due to weight loss in 10 to 20% of all cancer patients.

In the present disclosure, the "cachexia" may include, for example, cancer cachexia, AIDS cachexia, chronic obstructive pulmonary disease cachexia, multiple sclerosis cachexia, or congestive heart failure cachexia, but is not limited thereto, and specifically may be "cancer cachexia" or "anticancer agent-induced cachexia (cachexia induced by an anticancer agent)."

In the present disclosure, "anticancer chemotherapy" means a systemic treatment method for preventing the growth of or killing cancer cells by using drugs for cancer treatment, that is, anticancer agents, and "side effects of anticancer chemotherapy" means that, although the severity of each symptom varies depending on the type of anticancer chemotherapy, the main adverse events include loss of appetite, nausea, vomiting, diarrhea, stomatitis, bone marrow toxicity (leukopenia and granulocytopenia), skin disorders, alopecia, neurological disorders, interstitial pneumonia, liver disorders, kidney disorders, and heart disorders.

The pharmaceutical composition may each be formulated and used in the form of an orally administered formulation such as a powder, granule, tablet, capsule, suspension, emulsion, syrup, or aerosol, or in the form of an external preparation, suppository, or sterile injectable solution, according to a conventional method. Examples of carriers, excipients, and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil. Upon formulation, the composition is prepared using commonly used diluents or excipients such as fillers, extenders, binders, wetting agents, disintegrants, and surfactants. Solid formulations for oral administration include tablets, pills, powders, granules, and capsules, and such solid formulations are prepared by mixing the strain of the present disclosure or a culture thereof with at least one excipient, for example, starch, calcium carbonate, sucrose or lactose, gelatin, and the like. In addition to simple excipients, lubricants such as magnesium stearate and talc may also be used. Liquid formulations for oral administration include suspensions, internal solutions, emulsions, and syrups, and in addition to commonly used simple diluents such as water and liquid paraffin, various excipients such as wetting agents, sweeteners, flavoring agents, and preservatives may be included. Formulations for parenteral administration include sterile aqueous solutions, non-aqueous solvents, suspensions, emulsions, lyophilized formulations, and suppositories. As the non-aqueous solvents and suspensions, propylene glycol, polyethylene glycol, vegetable oils such as olive oil, injectable esters such as ethyl oleate, and the like may be used. As bases for suppositories, witepsol, macrogol, tween 61, cacao butter, laurin fat, glycerogelatin, and the like may be used.

The dosage of the pharmaceutical composition of the present disclosure will vary depending on the age, sex, and body weight of the subject to be treated, the particular disease or pathological condition to be treated, the severity of the disease or pathological condition, the route of administration, and the judgment of the prescribing physician. Determination of the dosage based on these factors is within the level of those skilled in the art, and administration may be performed once a day or may be divided into several administrations. The dosage is not intended to limit the scope of the present disclosure in any way.

The pharmaceutical composition of the present disclosure may be administered to mammals such as mice, livestock, and humans through various routes. All modes of administration may be contemplated, and for example, the composition may be administered by oral, rectal, intravenous, intramuscular, subcutaneous, intrauterine epidural, or intracerebrovascular injection. Since the strain of the present disclosure has little toxicity and few side effects, it is an agent that can be safely used even when taken over a long period for preventive purposes.

In the Examples of the present disclosure, in order to confirm the efficacy of Lactobacillus reuteri ATG-F4 in preventing, alleviating, or treating cachexia or side effects of anticancer chemotherapy, cachexia or side effects of anticancer chemotherapy were induced in cancer-induced mice by administration of anticancer agents, namely 5-FU, FOLFIRI, or FOLFOX, and Lactobacillus reuteri ATG-F4 according to the present disclosure was administered to these 5-FU-induced cachexia or side effects of anticancer chemotherapy models or FOLFIRI/FOLFOX-induced cachexia or side effects of anticancer chemotherapy models, and changes in the following symptoms of cachexia or side effects of anticancer chemotherapy were confirmed.

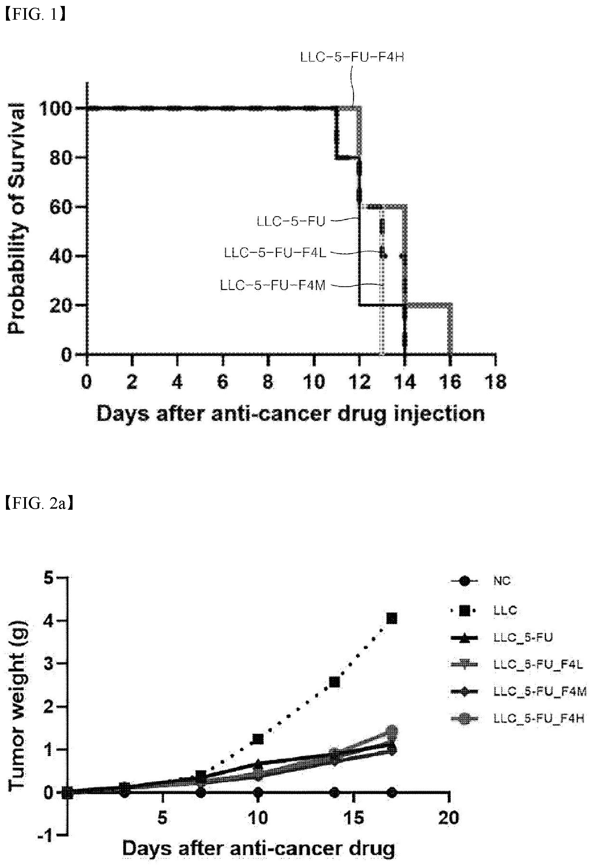

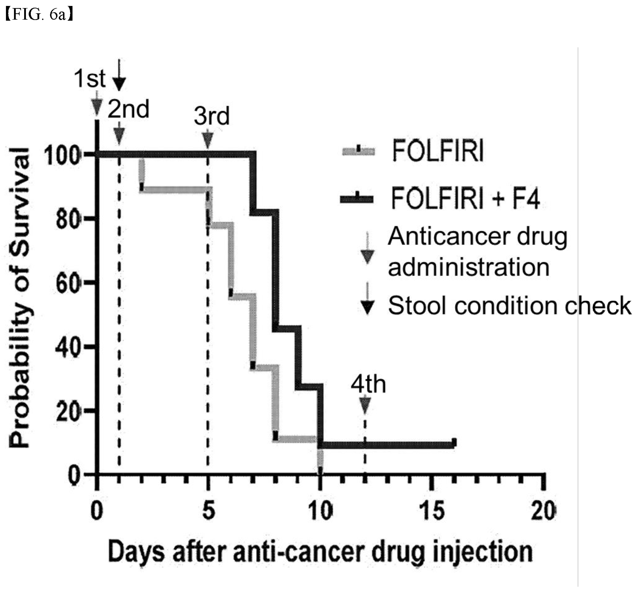

The composition of the present disclosure is characterized by increasing a survival rate reduced by administration of an anticancer agent. In the Examples of the present disclosure, as a result of administering Lactobacillus reuteri ATG-F4 to 5-FU- or FOLFIRI/FOLFOX-induced cachexia or side effects of anticancer chemotherapy models, it was confirmed that the survival rate increased and the survival period was prolonged (FIG. 1 and FIGS. 6a-6b).

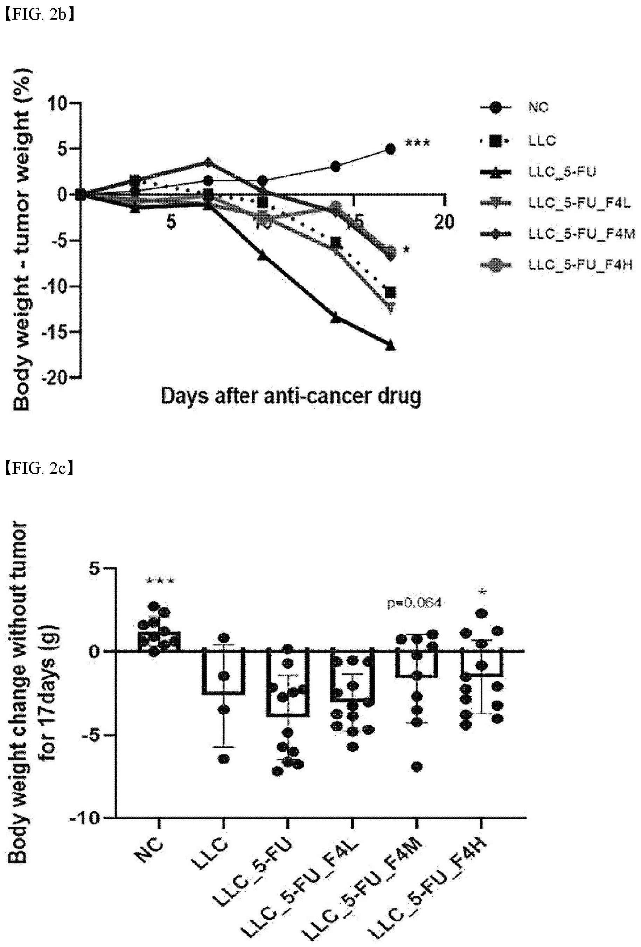

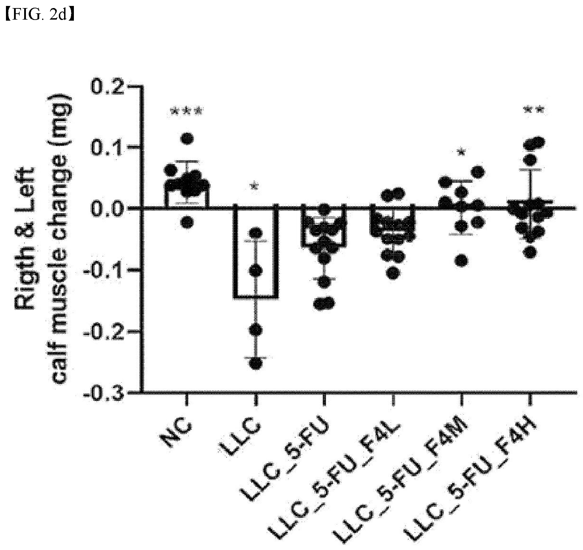

The composition of the present disclosure is characterized by increasing body weight, muscle mass, and fat mass reduced by administration of an anticancer agent. In the Examples of the present disclosure, as a result of administering Lactobacillus reuteri ATG-F4 to a 5-FU-induced cachexia or side effects of anticancer chemotherapy model, it did not affect the tumor growth inhibitory effect of 5-FU (FIG. 2a), but inhibited body weight loss caused by 5-FU (FIGS. 2b-2c) and also inhibited reductions in muscle mass and fat mass caused by 5-FU (FIGS. 2d-2e).

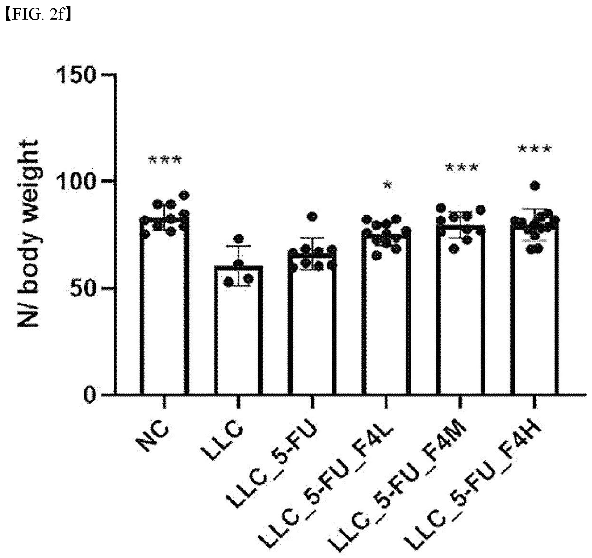

The composition of the present disclosure is characterized by increasing grip strength reduced by administration of an anticancer agent. In the Examples of the present disclosure, as a result of administering Lactobacillus reuteri ATG-F4 to a 5-FU-induced cachexia or side effects of anticancer chemotherapy model, grip strength reduced by 5-FU was increased (FIG. 2f).

The composition of the present disclosure is characterized by reducing inflammation increased by administration of an anticancer agent. The inflammation may be any one selected from muscle tissue inflammation, colon tissue inflammation, and blood inflammation.

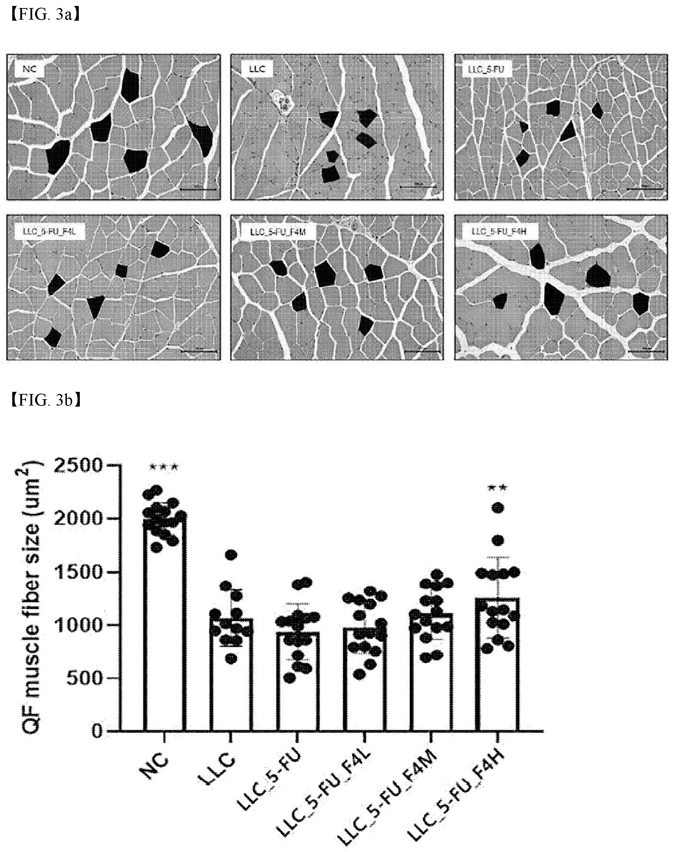

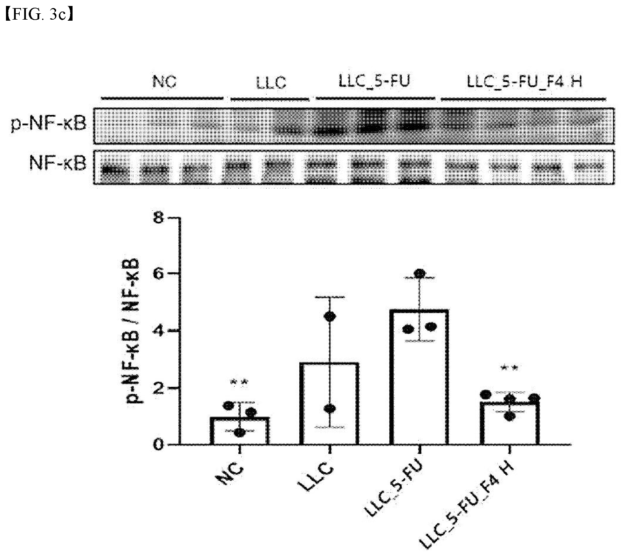

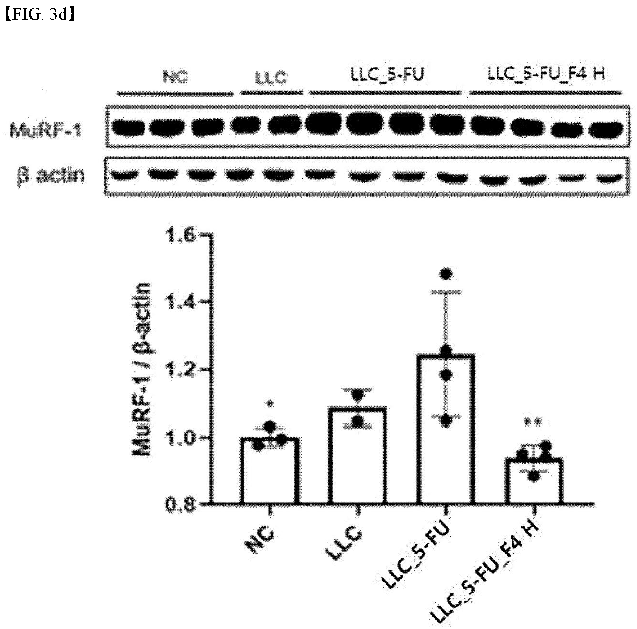

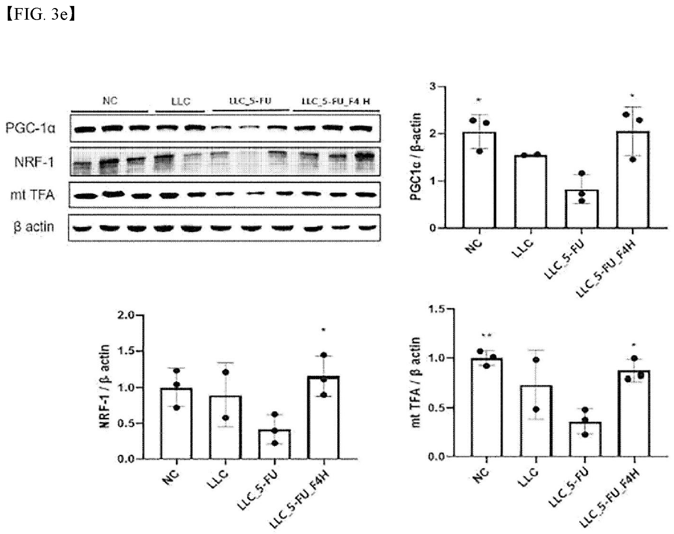

The composition of the present disclosure is characterized by reducing muscle tissue inflammation increased by administration of an anticancer agent, and more specifically, by inhibiting phosphorylation of NF-κB (nuclear factor-κB), which is an inflammation-related factor increased in muscle tissue by administration of an anticancer agent. In the Examples of the present disclosure, the composition of the present disclosure increased atrophied muscle fibers in muscle tissue, inhibited phosphorylation of NF-κB, reduced the muscle atrophy-related protein MuRF1, and increased mitochondria-related proteins PGC-1α, NRF-1, and mtTFA. Specifically, as a result of administering Lactobacillus reuteri ATG-F4 to a 5-FU-induced cachexia or side effects of anticancer chemotherapy model, the size of quadriceps femoris (QF) muscle fibers, which had been reduced by administration of the anticancer agent, increased (FIGS. 3a-3b), phosphorylation of NF-κB (p-NF-κB/NF-κB), which had been increased by administration of the anticancer agent, was inhibited (FIG. 3c), the muscle atrophy-related protein MuRF1, which had been increased by administration of the anticancer agent, was reduced (FIG. 3d), and mitochondria-related proteins PGC-1α, NRF-1, and mtTFA, which had been reduced by administration of the anticancer agent, increased (FIG. 3e).

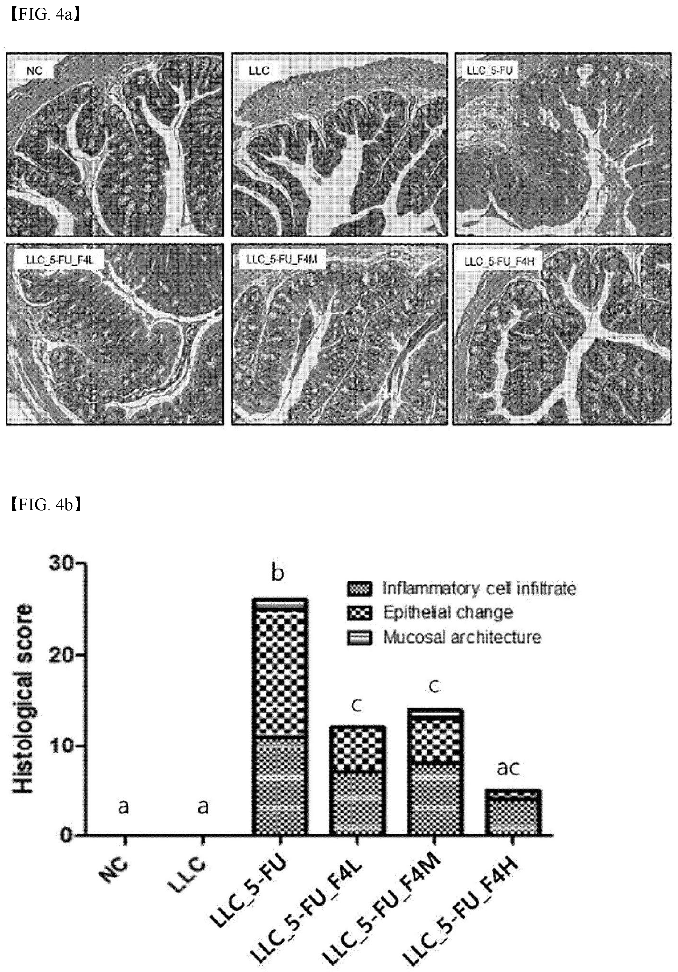

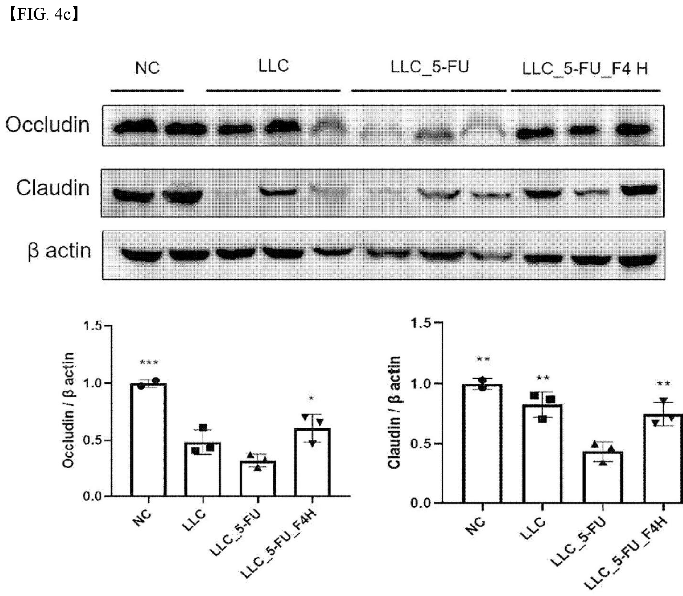

In addition, the composition of the present disclosure is characterized by reducing inflammation in colon tissue increased by administration of an anticancer agent, and more specifically, by reducing damage to colon tissue increased by administration of an anticancer agent. In the Examples of the present disclosure, damage to colon tissue (inflammatory cell infiltrate, epithelial cell changes, and changes in mucosal architecture in H&E staining) was confirmed in a 5-FU-induced cachexia or side effects of anticancer chemotherapy model caused by administration of an anticancer agent (FIG. 4a), and as a result of administering Lactobacillus reuteri ATG-F4, the damage to colon tissue was reduced (FIG. 4b). In addition, the composition of the present disclosure is characterized by restoring an expression level of occludin or claudin protein reduced in colon tissue by administration of an anticancer agent. In the Examples of the present disclosure, as a result of administering Lactobacillus reuteri ATG-F4 to a 5-FU-induced cachexia or side effects of anticancer chemotherapy model, it was confirmed that the expression levels of occludin or claudin protein, which are tight junction- or barrier-related proteins in colon tissue, were restored (FIG. 4c).

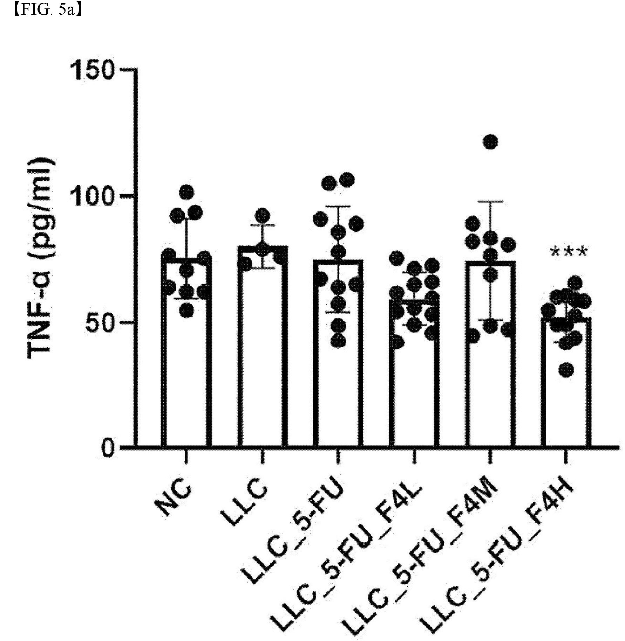

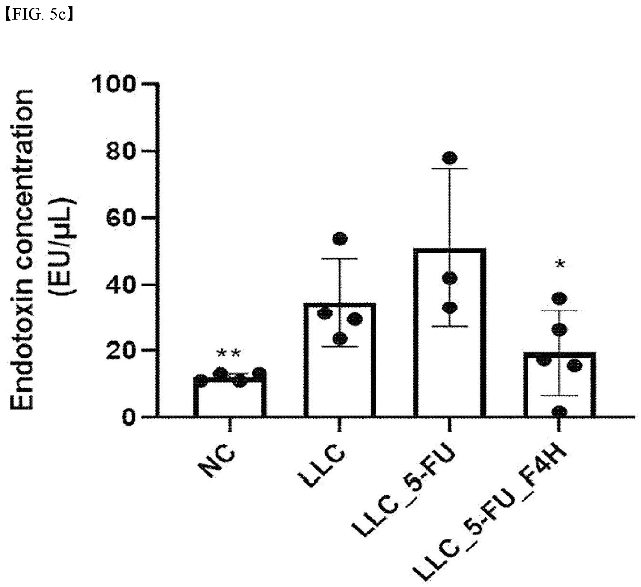

In addition, the composition of the present disclosure is characterized by reducing blood inflammation increased by administration of an anticancer agent. More specifically, the composition is characterized by reducing a blood concentration of TNF-α or IL-6 increased by administration of an anticancer agent, and/or reducing blood lipopolysaccharide (LPS) increased by administration of an anticancer agent. In the Examples of the present disclosure, as a result of administering Lactobacillus reuteri ATG-F4 to a 5-FU-induced cachexia or side effects of anticancer chemotherapy model, it was confirmed that the blood concentration of TNF-α and the blood concentration of IL-6, which are inflammatory cytokines increased by administration of an anticancer agent, were reduced (FIGS. 5a-5b). In addition, in the Examples of the present disclosure, as a result of administering Lactobacillus reuteri ATG-F4 to a 5-FU-induced cachexia or side effects of anticancer chemotherapy model, it was confirmed that blood influx of gut-derived lipopolysaccharide (LPS), which is an endotoxin, was reduced (FIG. 5c).

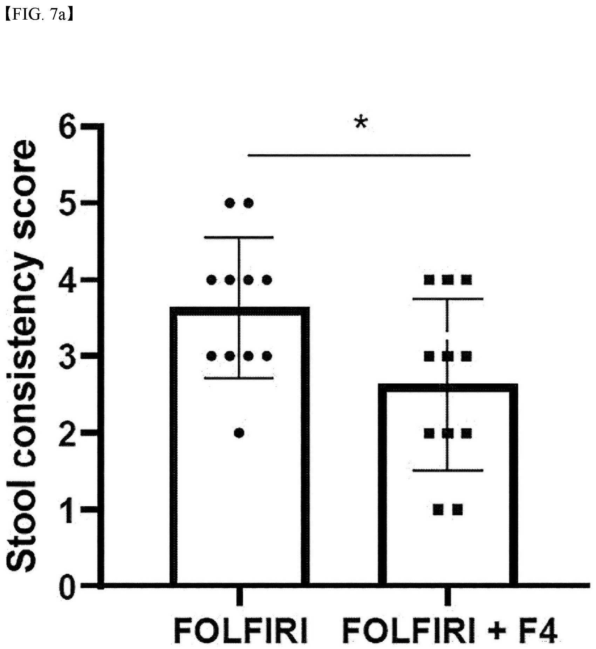

The composition of the present disclosure is characterized by reducing symptoms of diarrhea caused by administration of an anticancer agent. In the Examples of the present disclosure, as a result of administering Lactobacillus reuteri ATG-F4 to FOLFIRI- or FOLFOX-induced cachexia or side effects of anticancer chemotherapy models, it was confirmed that stool consistency score values were lowered, thereby reducing symptoms of diarrhea (FIGS. 7a-7b).

Anticancer agents are broadly classified according to their mechanisms of action into cytotoxic chemotherapeutic agents, targeted anticancer agents, and immunotherapeutic anticancer agents. Among these, cytotoxic chemotherapeutic agents exert anticancer effects by directly attacking cancer cells that divide more rapidly than normal cells, and include alkylating agents (oxaliplatin, cisplatin, cyclophosphamide, ifosfamide, bendamustine, melphalan, carboplatin, busulfan, dacarbazine, and temozolomide); antimetabolites (fluorouracil, capecitabine, doxifluridine, tegafur, cytarabine, azacitidine, decitabine, enocitabine, methotrexate, pemetrexed, pralatrexate, cladribine, clofarabine, fludarabine, and mercaptopurine); DNA topoisomerase inhibitors (irinotecan, doxorubicin, daunorubicin, epirubicin, idarubicin, mitoxantrone, etoposide, and topotecan); microtubule inhibitors (cabazitaxel, paclitaxel, docetaxel, vinblastine, vincristine, and vinorelbine); and others (bleomycin, hydroxyurea, and mitomycin C), and these chemotherapeutic anticancer agents mainly have similar mechanisms of action involving DNA damage or inhibition of DNA synthesis.

As described above, in the Examples of the present disclosure, it was confirmed that the Lactobacillus reuteri ATG-F4 strain of the present disclosure was effective in preventing, alleviating, or treating cachexia or side effects of anticancer chemotherapy caused by administration of 5-FU alone, and was also effective in preventing, alleviating, or treating cachexia or side effects of anticancer chemotherapy caused by administration of the combined anticancer agents FOLFIRI and FOLFOX (both of which are 5-FU-based anticancer agents). Therefore, the Lactobacillus reuteri ATG-F4 strain of the present disclosure is effective in preventing, alleviating, or treating cachexia or side effects of anticancer chemotherapy caused by administration of anticancer agents, and in particular, in view of the types of mechanisms of action of cytotoxic chemotherapeutic agents, is expected to be capable of reducing side effects of anticancer agents related to alkylating agents, antimetabolites, and DNA topoisomerase inhibitors.

In addition, since the Lactobacillus reuteri ATG-F4 strain of the present disclosure also exhibited effects on cachexia or side effects of anticancer chemotherapy caused by administration of the combined anticancer agents FOLFIRI and FOLFOX, the Lactobacillus reuteri ATG-F4 strain of the present disclosure is expected to reduce side effects of anticancer agents associated with combination anticancer chemotherapy in which two or more agents are simultaneously used to increase anticancer effects.

Targeted anticancer agents selectively attack cancer cells, and immunotherapeutic anticancer agents operate on the principle that immune cells better recognize and attack cancer cells. Although these agents have the advantage of causing fewer side effects than cytotoxic chemotherapeutic agents, they have disadvantages in that they take a long time to exhibit therapeutic effects and resistance may develop, and thus are often used together with cytotoxic chemotherapeutic agents. Considering that cytotoxic chemotherapeutic agents directly attack and kill cancer cells and also attack normal cells, thereby causing side effects, symptoms of cachexia and side effects of anticancer agents appear to be more attributable to cytotoxic chemotherapeutic agents than to targeted anticancer agents or immunotherapeutic anticancer agents. That is, the Lactobacillus reuteri ATG-F4 strain of the present disclosure is also expected to alleviate or reduce cachexia symptoms and side effects of anticancer agents induced by use of cytotoxic chemotherapeutic agents together with (or simultaneously with) targeted anticancer agents and/or immunotherapeutic anticancer agents, and thus is also expected to have efficacy in preventing, alleviating, or treating resulting cachexia or side effects of anticancer chemotherapy.

Hereinafter, the present disclosure will be described in detail with reference to Examples and Experimental Examples. However, the following Examples and Experimental Examples are provided only for illustrating the present disclosure, and the scope of the present disclosure is not limited thereto.

### Experimental Example 1: Increase in Survival Rate and Alleviation of Cachexia Symptoms in 5-FU-Induced Cachexia Symptoms

### [Experimental Methods]

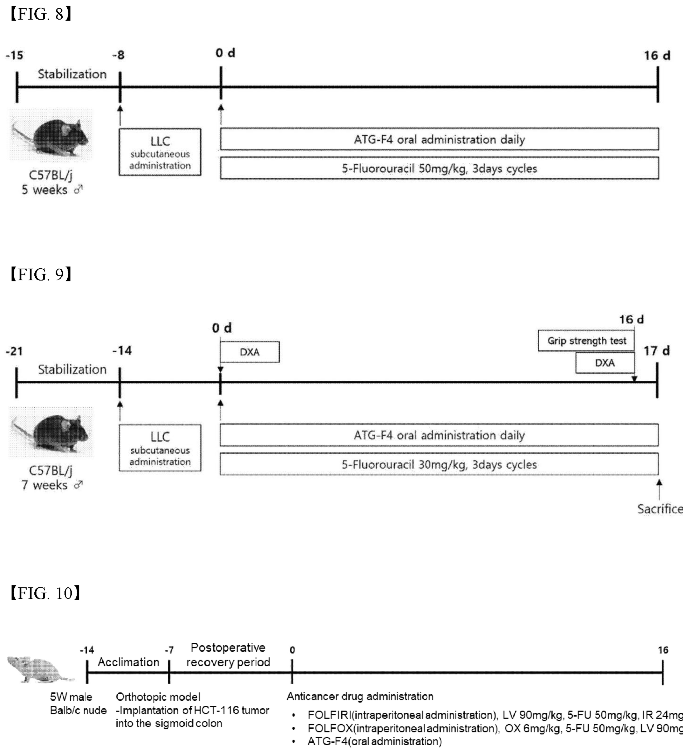

Prior to all animal experiments conducted in this Experimental Example, the animal experiments were approved by the Institutional Animal Care and Use Committee (IACUC) of ATOGEN Co., Ltd. in accordance with appropriate procedures (Approval No.: ATG-IACUC-RDSP-220321), and guidance for ethical animal experimentation was provided. The animals used in this Experimental Example were 5-week-old (FIG. 8) or 7-week-old (FIG. 9) C57BL/j mice, which were purchased from Raonbio (Seoul, Korea) and used in the experiments. Lewis lung carcinoma cells (LLC, ATCC, Manassas, VA, USA) were cultured in DMEM (Dulbecco's modified Eagle's medium) supplemented with 10% FBS (fetal bovine serum).

The mice were housed in an animal room maintained at a constant room temperature of 23 ± 2°C, with controlled temperature and humidity and a 12:12 light cycle. The mice were allowed an acclimation period of at least 7 days to adapt to the new environment and feed, and were provided with water and rodent chow (Purina) ad libitum.

After the acclimation period, 0.1 mL of sterile normal saline was subcutaneously injected into the right flank of 10 mice in the normal control group. The remaining mice were subcutaneously inoculated with 5 × 10⁵ cells of LLC cells. Fourteen days after inoculation, the LLC-treated mice were divided into five groups so that the tumor sizes were similar.

First, the experimental schedule and group setting for the survival rate-related experiment were established as shown in FIG. 8 and Table 2. ATG-F4 was mixed with PBS and orally administered at three doses (1.0 x 10⁸, 1.0 x 10⁹, and 1.0 x 10¹⁰ CFU), and 50 mg/kg of 5-fluorouracil (5-FU) was intraperitoneally injected in a 3-day cycle.

**[Table 2]**

| Group | | | ATG-F4 | Number (n) |
|---|---|---|---|---|
| 1 | LLC + 5-FU | LLC_5-FU | Vehicle | 5 |
| 2 | LLC + 5-FU + ATG-F4 low | LLC_5-FU_F4L | 1.0 x 10⁸ CFU/day | 5 |
| 3 | LLC + 5-FU + ATG-F4 Medium | LLC_5-FU_F4M | 1.0 x 10⁹ CFU/day | 5 |
| 4 | LLC + 5-FU + ATG-F4 High | LLC_5-FU_F4H | 1.0 x 10¹⁰ CFU/day | 5 |

Second, the experimental schedule and group setting for the ATG-F4 efficacy evaluation-related experiment were established as shown in FIG. 9 and Table 4. ATG-F4 was mixed with PBS and orally administered at three doses (4.0 x 10⁸, 4.0 x 10⁹, and 4.0 x 10¹⁰ CFU), and 30 mg/kg of 5-fluorouracil (5-FU) was intraperitoneally injected in a 3-day cycle.

**[Table 4]**

| Group | | | ATG-F4 | Number (n) |
|---|---|---|---|---|
| 1 | Normal Control | NC | Vehicle | 10 |
| 2 | LLC only | LLC | Vehicle | 4 |
| 3 | LLC + 5-FU | LLC_5-FU | Vehicle | 12 |
| 4 | LLC + 5-FU + ATG-F4 low | LLC_5-FU_F4L | 4.0 x 10⁸ CFU/day | 13 |
| 5 | LLC + 5-FU + ATG-F4 Medium | LLC_5-FU_F4M | 4.0 x 10⁹ CFU/day | 10 |
| 6 | LLC + 5-FU + ATG-F4 High | LLC_5-FU_F4H | 4.0 x 10¹⁰ CFU/day | 13 |

Tumor volume was measured three-dimensionally using a caliper and then calculated using the following equation: tumor volume = (height × width × length) × 0.5 cm³. Whole-body fat-free mass and calf fat-free mass were measured using DXA (model iNSiGHT VET DXA, Osteosys, Korea). The grip strength test was performed on the day before necropsy. On the day of necropsy, blood was collected from the posterior vena cava after CO₂ euthanasia. The collected blood was centrifuged at 4000 rpm for 10 minutes to obtain serum. Quadriceps femoris (QF) was isolated from the calf of the right lower body and weighed. The colon was collected and stored in a -70°C freezer until analysis.

### [Analysis Method]

### 1. Western Blot Analysis

QF muscle or colon tissue was placed in RIPA buffer (0.5 M Tris-HCl, pH 7.4, 1.5 M NaCl, 2.5% deoxycholic acid, and 10% NP-40) containing a protease inhibitor cocktail (Millipore, USA), and finely cut with scissors. Thereafter, the mixture was centrifuged at 14,000 rpm at 4°C for 10 minutes, and the supernatant was collected. The protein concentration of the supernatant was measured using a BCA assay (Thermo Fisher, USA). Protein extracts (40 µg) were separated using an 8% or 10% polyacrylamide mini-gel and then transferred onto a PVDF membrane (Bio-Rad, USA). The transferred PVDF membrane was immersed in SuperBlock (PBS) Blocking Buffer (pH 7.4) containing Kathon^{™} Antimicrobial Agent and primary antibodies against MuRF1 (1:1000; Mybioscience), p-NF-κB (1:1000; CST), NF-κB (1:1000; CST), PGC-1α (1:1000; Abcam), mtTFA (1:1000; Abcam), NRF-1 (1:1000; Abcam), occludin (1:1000; Thermo Fisher), claudin (1:1000; Thermo Fisher), and β-actin (1:1000; Cell Signaling), followed by overnight incubation at 4°C. The PVDF membrane was washed four times with 0.1% Tween TBS, and then placed in 0.1% Tween TBS buffer containing 3% BSA (Bovogen, USA) and incubated with a goat anti-rabbit IgG HRP-conjugated secondary antibody (Bio-Rad, USA) at room temperature for 1 hour. After thorough washing with 0.1% Tween TBS, the immunostained bands were detected using ECL (Bio-Rad, USA). Detection of the target proteins was performed using a ChemiDoc^{™} Imaging System (Bio-Rad, USA), and band intensity was quantified using Image Lab^{™} software (Bio-Rad, USA).

### 2. Grip Strength Test

On the day before necropsy, grip strength (Biosep, Bio GS3) was measured. After allowing all four paws to be placed on a metal rail, the tail was pulled with a constant force to measure tension. Each animal was measured nine times.

### 3. Histology

After the colon and quadriceps femoris (QF) muscle were separated, they were fixed by immersion in 10% formalin at room temperature for at least 48 hours. Using the fixed tissues, specimens for histopathological examination were prepared through general tissue processing procedures including trimming, dehydration, paraffin embedding, and sectioning, followed by Hematoxylin & Eosin (H&E) staining. Histopathological changes were observed through H&E slide images magnified 400 times using an optical microscope (Olympus BX53, Japan). Muscle fiber size was quantified on the H&E slides. Three regions were selected from one tissue slide, and a total of 30 or more muscle fibers were quantified and compared. Colon tissue was quantified using three inflammation-related indicators, namely inflammatory cell infiltrate, epithelial change, and mucosal architecture.

### 4. Endotoxin Analysis

Serum endotoxin levels were analyzed using ToxinSensor^{™} Chromogenic LAL Endotoxin Assay Kit (Cat.No.: L00350).

### 5. Cytokine Analysis

Serum IL-6 and TNF-α levels were measured using a commercial kit (mouse IL-6 ELISA max standard set, BioLegend).

### [Results]

Referring to FIG. 1, it was confirmed that administration of ATG-F4 at 1.0 x 10¹⁰ CFU prolonged the survival period by 2 days.

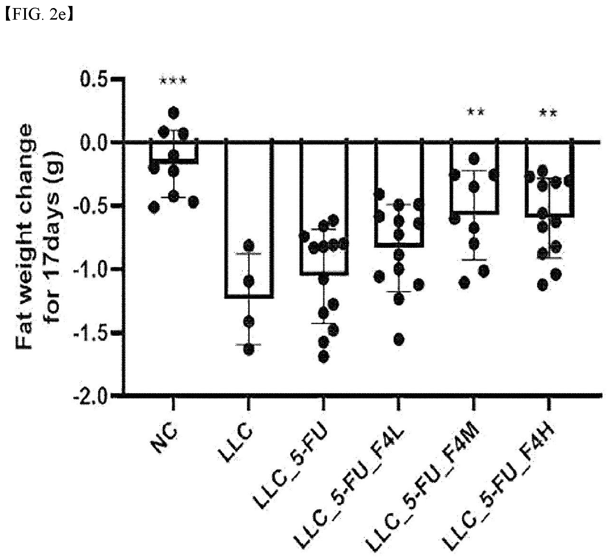

Referring to FIGS. 2a to 2f, ATG-F4 did not affect the anticancer effect of 5-FU (FIG. 2a), but inhibited body weight loss caused by the anticancer agent (FIGS. 2b and 2c), and increased calf muscle mass (FIG. 2d), fat mass (FIG. 2e), and grip strength (FIG. 2f).

Referring to FIGS. 3a to 3e, in QF muscle tissue, ATG-F4 increased muscle fiber size reduced by 5-FU (FIGS. 3a and 3b), inhibited an inflammation-related protein (p-NF-κB/NF-κB) in QF muscle (FIG. 3c), inhibited a muscle atrophy-related protein (MuRF1) (FIG. 3d), and activated mitochondria-related proteins (PGC-1α, NRF-1, and mtTFA) (FIG. 3e).

Referring to FIGS. 4a to 4c, in colon tissue, ATG-F4 inhibited inflammation increased by 5-FU (FIGS. 4a and 4b), and increased expression of barrier-related proteins (occludin and claudin) in colon tissue (FIG. 4c).

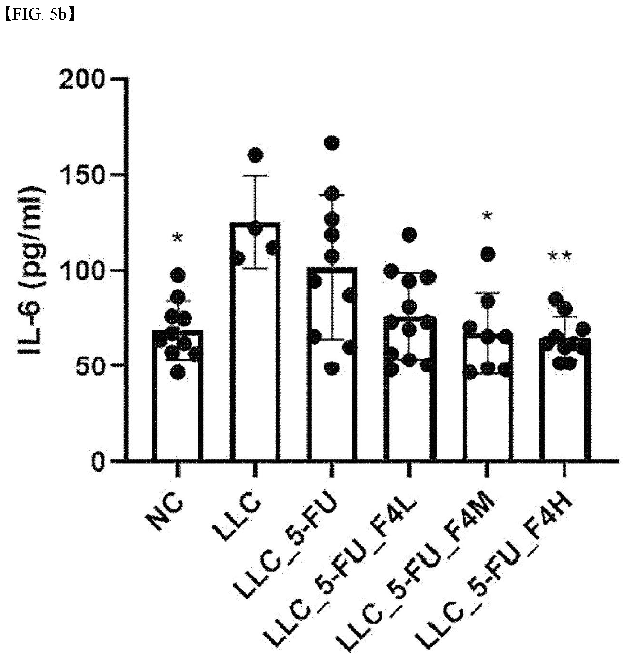

Referring to FIGS. 5a to 5c, ATG-F4 inhibited the inflammatory cytokines TNF-α and IL-6 in blood (FIGS. 5a and 5b), and reduced blood influx of gut-derived LPS (FIG. 5c).

### Experimental Example 2: Confirmation of Increased Survival Rate and Improvement of Diarrhea Symptoms in FOLFIRI- and FOLFOX-Induced Cachexia Symptoms

### [Experimental Methods]

Prior to all animal experiments conducted in this Experimental Example, the animal experiments were approved by the Institutional Animal Care and Use Committee (IACUC) of ATOGEN Co., Ltd. in accordance with appropriate procedures (Approval No.: ATG-IACUC-RDSP-230427), and guidance for ethical animal experimentation was provided. The animals used in this Experimental Example were 5-week-old Balb/c nude mice, which were purchased from Raonbio (Seoul, Korea) and used in the experiments.

HCT-116 cells (human colon cancer cell line, Korean Cell Line Bank) were cultured in DMEM (Dulbecco's modified Eagle's medium) supplemented with 10% FBS (fetal bovine serum).

The mice were housed in an animal room maintained at a constant room temperature of 23 ± 2°C, with controlled temperature and humidity and a 12:12 light cycle. The mice were allowed an acclimation period of at least 7 days to adapt to the new environment and feed, and were provided with water and rodent chow (Purina) ad libitum.

The experimental schedule and group setting for the survival rate-related experiment were established as shown in FIG. 10 and Table 6. After the acclimation period, 1.0 x 10⁶ cells of HCT-116 cells were subcutaneously administered into the flanks of the mice, and the tumors were allowed to grow for 10 days. The tumors were cut to a size of 1.5 mm in width × length × height and fixed to the mouse sigmoid colon using surgical sutures.

At this time, the sigmoid colon was slightly scratched with a razor blade so that the tumor could be well engrafted.

After the tumor was transplanted into the sigmoid colon, a recovery period of 7 days was allowed. Thereafter, FOLFIRI (Leucovorin 90mg/kg, 5-FU 50mg/kg, Irinotecan 24mg/kg) or FOLFOX (Oxaliplatin 6mg/kg, 5-FU 50mg/kg, Leucovorin 90mg/kg) was intraperitoneally administered, and ATG-F4 at 4.0 x 10¹⁰ CFU was orally administered daily. Survival rate was observed after administration. Stool consistency score (evaluation of improvement of diarrhea symptoms, Table 7) was assessed at 30 minutes after the second administration of each of FOLFIRI and FOLFOX.

**[Table 6]**

| Group | | ATG-F4 | Number (n) |
|---|---|---|---|
| 1 | Cancer + FOLFIRI | Vehicle | 11 |
| 2 | Cancer + FOLFIRI + ATG-F4 | 4.0x 10¹⁰CFUday | 11 |
| 3 | Cancer + FOLFOX | Vehicle | 11 |
| 4 | Cancer + FOLFOX + ATG-F4 | 4.0x 10¹⁰CFUday | 11 |

**[Table 7]**

| Score | Status | Details |
|---|---|---|
| 0 | Normal | Hard stool with no moisture |
| 1 | Slightly soft stool | Stool with low moisture content and reduced hardness |
| 2 | Soft stool | Stool with some moisture; when pressed with forceps, it retains its shape, and the forceps sink in |
| 3 | Soft stool with mild diarrhea | Stool with high moisture content; when pressed with forceps, it retains its shape but becomes mushy |
| 4 | Diarrhea | Stool with high moisture content; when pressed with forceps, it does not retain its shape and becomes mushy |
| 5 | Severe watery diarrhea | Stool with high moisture content and no shape from the time of excretion |

### [Results]

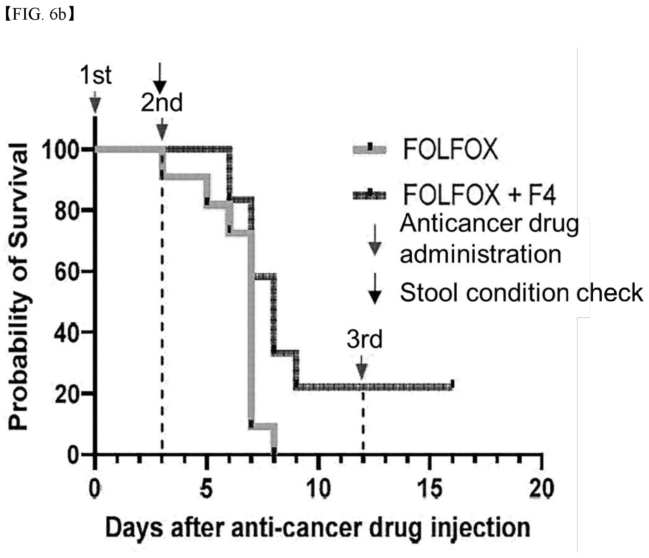

Referring to FIGS. 6a and 6b, it was confirmed that administration of ATG-F4 at 1.0 x 10¹⁰ CFU significantly prolonged the survival period of the FOLFIRI and FOLFOX groups.

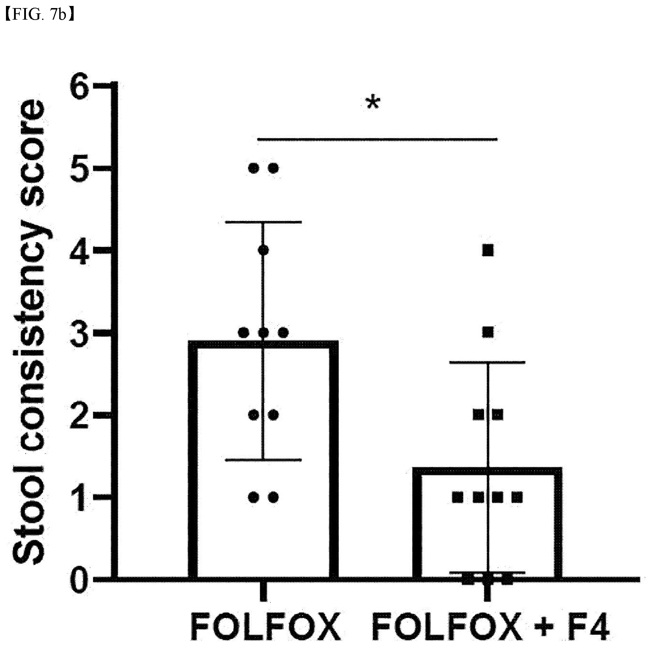

Referring to FIGS. 7a and 7b, it was confirmed that administration of ATG-F4 at 1.0 x 10¹⁰ CFU lowered stool consistency score values occurring in the FOLFIRI and FOLFOX groups, thereby reducing symptoms of diarrhea to a level similar to that of the normal group.

Although specific exemplary embodiments relating to a composition for preventing, alleviating, or treating cachexia or side effects of anticancer chemotherapy, comprising Lactobacillus reuteri ATG-F4 strain according to an exemplary embodiment of the present disclosure, have been described above, it is obvious that various modifications may be made without departing from the scope of the present disclosure.

Accordingly, the scope of the present disclosure should not be limited to the described exemplary embodiments, but should be defined not only by the claims set forth below but also by equivalents thereto.

That is, the above-described exemplary embodiments should be understood in all respects as illustrative and not restrictive, and the scope of the present disclosure is indicated by the claims set forth below rather than by the detailed description, and all changes or modified forms derived from the meaning and scope of the claims and equivalent concepts thereof should be construed as being included within the scope of the present disclosure.

### [Industrial Applicability]

The present disclosure provides a composition for preventing, alleviating, or treating cachexia or side effects of anticancer chemotherapy, comprising Lactobacillus reuteri ATG-F4 strain as an active ingredient.

### [Accession Number]

Name of Depositary Institution: Korean Collection for Type Cultures (KCTC)
Accession No.: KCTC13717BP
Date of Deposit: November 15, 2018

## Claims

1. A composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

2. A pharmaceutical composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

3. A food composition for preventing, alleviating, or treating cachexia, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

4. A composition for preventing, alleviating, or treating side effects of anticancer chemotherapy, comprising, as an active ingredient, at least one selected from the group consisting of a strain of Lactobacillus reuteri ATG-F4 *(Lactobacillus reuteri* ATG-F4, accession No. KCTC13717BP), cells of the strain, a culture of the strain, an extract of the culture, a concentrate of the culture, and a dried product of the culture.

5. The composition according to any one of claims 1 to 4, wherein the composition increases a survival rate reduced by administration of an anticancer agent.

6. The composition according to any one of claims 1 to 4, wherein the composition increases a fat mass reduced by administration of an anticancer agent.

7. The composition according to any one of claims 1 to 4, wherein the composition increases grip strength reduced by administration of an anticancer agent.

8. The composition according to any one of claims 1 to 4, wherein the composition reduces inflammation increased by administration of an anticancer agent.

9. The composition according to any one of claims 1 to 4, wherein the composition reduces symptoms of diarrhea caused by administration of an anticancer agent.
